# DEMANDE DE BREVET EUROPEEN

(11) **EP 0 673 651 A1**
(43) Date de publication de la demande: **27.09.1995**
(21) Numéro de dépôt: 95400634.2
(22) Date de dépôt: 22.03.1995
(51) Int. Cl.: A61K 33/06, A61K 35/02

(54) **Nouvelle composition médicamenteuse à base d'argile**

(30) Priorité: 23.03.1994 FR 9403428
(71) Demandeur: THIRIEZ, Florence, F-75003 Paris (FR)
(72) Inventeur: THIRIEZ, Florence, F-75003 Paris (FR)
(74) Mandataire: Gallochat, Alain

(57) **Abrégé**

La présente invention concerne une nouvelle composition médicamenteuse à base d'argile.

Parmi les diverses argiles connues telles que la smectite, la kaolinite, l'attapulgite et l'illite, cette dernière variété est préférée à raison d'au moins 50 % en poids de la partie argileuse de la composition médicamenteuse.

La composition médicamenteuse selon l'invention est adaptée à un usage externe et/ou interne pour le traitement de diverses infections.

## Description

La présente invention concerne une nouvelle composition médicamenteuse à base d'argile.

Diverses argiles ont jusqu'à présent été utilisées en thérapeutique ; il s'agit plus particulièrement de certaines variétés d'argiles, à savoir les smectites, les kaolinites et les attapulgites. Les deux premières variétés (smectites, kaolinites) sont constituées de feuillets, à trois couches pour les smectites (une couche d'octaèdres à coeur d'aluminium prise en sandwich entre deux couches de tétraèdres à coeur de silicium) et à deux couches pour les kaolinites (une couche d'octaèdres à coeur d'aluminium et une couche de tétraèdres à coeur de silicium) ; s'agissant des attapulgites, la structure est fibreuse et non plus en feuillets.

Sur le plan thérapeutique, les kaolinites sont connues depuis très longtemps et sont surtout prescrites pour les problèmes inflammatoires et ulcéreux de la sphère digestive auxquels leur pouvoir couvrant et leur bonne absorption apportent une amélioration symptomatique rapide.

Les smectites sont utilisées depuis moins longtemps ; ces argiles présentent de nombreuses substitutions au niveau du silicium et de l'aluminium du réseau par des ions de valence inférieure conférant aux feuillets une charge globalement négative. Ceci attire dans l'espace interfoliaire des cations de compensation dont la faible liaison les rend très facilement interchangeables, cette propriété conférant aux smectites un pouvoir d'adsorption très élevé pouvant s'exercer vis à vis des ions du milieu, des molécules organiques aussi bien que des virus ; les propriétés catalytiques de surface (surface spécifique : 30 à 50 m²/g, développement réel 700 m²/g) semblent également avoir une relation avec l'activité thérapeutique des smectites, à savoir le traitement per os symptomatique des colopathies fonctionnelles et les gastro-entérites. Il y a toutefois lieu de signaler certaines observations cliniques d'aggravation infectieuse en usage externe.

A titre comparatif, les attapulgites, qui sont de structure fibreuse, présentent une surface spécifique de l'ordre de 140 m²/g, et sont utilisées notamment pour renforcer les défenses de la muqueuse gastro-intestinale face à une agression.

La nouvelle variété d'argile choisie pour son activité thérapeutique présente des propriétés tout à fait intéressantes et comparables, voire supérieures, à celles des smectites, des kaolinites et des attapulgites, sans en présenter les inconvénients.

De façon plus précise, la présente invention concerne une nouvelle composition médicamenteuse à base d'illite.

Préférentiellement l'illite sera utilisée pure, ou représentera au moins 50% en poids de la partie argileuse de la composition médicamenteuse ; de plus et en raison de certains problèmes, notamment de toxicité, la présente composition sera pratiquement exempte d'attapulgite.

L'illite est une variété d'argile connue, du type à trois couches (une couche d'octaèdres à coeur d'aluminium prise en sandwich entre deux couches de tétraèdres à coeur de silicium) ; dans cette variété, les ions Fe et Mg peuvent remplacer Al dans la couche alumineuse et Al peut remplacer Si dans la couche siliceuse. En outre, les charges négatives sont équilibrées par des ions K qui unissent les feuillets les uns aux autres, une distance de l'ordre de 10 A séparant deux feuillets contigüs ; dans la présente description, le terme illite couvre de façon générique les différentes variétés d'illite, les illites présentant la structure qui vient d'être décrite constituant une famille, de même qu'il existe une famille des smectites, une famille des kaolinites et une famille des attapulgites.

De même que la smectite, l'illite a un bon pouvoir absorbant puisque ces argiles absorbent respectivement 20 et 25% de liquide par rapport à leur masse ; à titre de comparaison, l'attapulgite absorbe environ 40% de liquide. En revanche, ces variétés se distinguent par leur pouvoir adsorbant puisque la C.E.C. (Capacité d'Echange Cationique) de la smectite est de l'ordre de 40 à 80 meq/100 g (certaines variétés pouvant aller jusqu'à 120 meq/100 g) alors qu'elle n'est que de 15 à 20 meq/100 g pour l'attapulgite et de 8 à 10 meq/100 g pour l'illite s'agissant du pouvoir couvrant, il est de 80 m²/g pour la smectite, et seulement de 10 à 30 m²/g pour l'illite.

L'illite n'a, à ce jour, fait l'objet d'aucune application thérapeutique avec des doses correspondantes, c'est-à-dire à des doses supérieures ou égales à 150 mg/kg de poids corporel/jour.

La demanderesse a pu démontrer, et c'est ce qui fait l'objet de la présente demande, que l'illite avait des propriétés thérapeutiques particulièrement intéressantes en dépit d'un pouvoir adsorbant moyen, alors que, traditionnellement, ces propriétés sont liées au fort pouvoir adsorbant des argiles utilisés en thérapeutique. Il est en effet important de noter que c'est cette capacité d'adsorption qui est à ce jour reconnue comme la plus intéressante sur le plan curatif, puisque c'est elle qui permet la fixation, et donc la neutralisation, des toxines ou des alcaloïdes.

L'illite trouvera particulièrement une application dans le traitement des gastro-entérites en raison notamment de son fort pouvoir absorbant pour l'eau en excès et de sa richesse en potassium dont l'apport est précieux dans cette affection, mais trouvera aussi une série d'applications en usage externe.

A titre d'exemples non limitatifs, l'illite pourra être utilisée de la façon suivante :
- En usage externe :
   1. Traitement des infections utérines.
      Un cataplasme d'une pâte argileuse composée à 50% minimum d'illite, est préparé, d'une épaisseur de 1,5 à 2 cm et est appliqué sur le bas ventre, en contact direct avec la peau. La patiente restera de préférence allongée et son cataplasme sera renouvelé jusqu'à disparition des pertes nauséabondes. Ce traitement sera repris en cas de retour des symptômes, ou pour consolider la disparition de ces derniers, afin de garantir l'évacuation totale de tout germe ou toxine.
   2. Traitement des coupures.
      Dès l'effraction cutanée et dans la mesure où la blessure est propre, on appliquera de l'argile en poudre composée à 50% minimum d'illite comme suit : laisser tomber l'argile en pluie directement sur la plaie en fonction du saignement ; les argiles possèdent en effet des propriétés hémostatiques et on laissera tomber suffisamment de poudre pour "boire" le sang. Ce traitement sera arrêté dès qu'il y aura en surface de la poudre sèche. En cas de ré-émergence du sang, le traitement sera repris jusqu'à ce qu'il y ait sur la plaie un petit monticule d'argile et de sang séché : bien poudrer à sec la surface. Ce bouchon tombera spontanément et on verra alors apparaître une fine cicatrice blanche bien rectiligne sans avoir recours aux points de suture. Dans certains cas il faudra avoir recours à des applications larges et épaisses de pâte d'argile si les tissus avoisinant la plaie venaient à gonfler ; ce cataplasme serait à renouveler toutes les deux heures jusqu'à ce que la plaie soit propre. Le traitement avec la poudre d'argile pourra alors être repris.
   3. Traitement des otites aiguës.
      Faire une pâte épaisse avec de l'eau et de l'argile à 50% minimum d'illite et remplir l'espace rétro-pavillonnaire de l'oreille malade, c'est-à-dire l'espace situé derrière l'oreille entre le crâne et le pavillon de l'oreille ; en cas d'otite chronique, plusieurs applications peuvent être nécessaires, qui provoqueront un écoulement de l'oreille faisant sortir de cette dernière toute l'infection.
- En usage externe et interne :
   1. Traitement des rhumatismes.
      Avec une argile composée à 50% minimum d'illite, faire une pâte épaisse et préparer un cataplasme large, débordant la zone douloureuse, et épais, de l'ordre de 2 cm, qui sera directement appliqué sur la partie malade. Le cataplasme sera gardé toute la nuit et le traitement externe sera complété par une prise interne journalière d'une cuillère à café d'argile en poudre dans un verre d'eau. La durée du traitement et le rythme des applications seront établis en fonction de l'intensité des douleurs rhumatismales et de la gêne dans la mobilité du patient.
   2. Traitement des cystites.
      La valeur d'une cuillerée à soupe de poudre sèche d'argile composée d'au minimum 50% d'illite est versée en pluie dans un verre d'eau. Le patient absorbera ce mélange à la survenue des brûlures urinaires et attendra la survenue des douleurs ultérieures pour absorber une éventuelle deuxième dose. Ce traitement per os peut être complété par voie locale externe en introduisant dans le vagin, jusqu'à l'espace libre avoisinant le col de l'utérus, un petit caillou rond d'argile à base d'illite.
- En usage interne :
   Les épisodes diarrhéiques seront traités par des prises d'argile composée au minimum de 50% d'illite, à raison de 300 mg d'argile par kilo du patient ; dans le cas de récidives, il sera conseillé un traitement d'entretien à raison d'une cuillère à café deux fois par jour. L'effet a été constaté quelle que soit l'étiologie (stress, origine alimentaire, virus, bactéries ou certaines parasitoses).

Outre les avantages thérapeutiques de l'illite tels qu'ils ont été signalés dans la présente description, il est important de noter que la composition médicamenteuse faisant l'objet de la présente invention peut être réalisée à faible coût et dans pratiquement tous les pays, y compris les pays en voie de développement, en raison de la répartition très large des argiles contenant de l'illite dans l'écorce terrestre.

## Revendications

1. Composition médicamenteuse à base d'argile caractérisée en ce que ladite argile est de l'illite pratiquement exempte d'attapulgite.

2. Composition selon la revendication 1 caractérisée en ce que ladite illite est présente à raison de 50 % au moins en poids de la partie argileuse de ladite composition.

3. Composition selon l'une des revendications 1 ou 2 caractérisée en ce qu'elle se présente sous forme de cataplasme à usage externe.

4. Composition selon l'une des revendications 1 ou 2 caractérisée en ce qu'elle se présente sous forme de poudre.

5. Composition selon la revendication 4 caractérisée en ce que ladite poudre est destinée à un usage per os.

6. Composition selon l'une des revendications 4 ou 5 caractérisée en ce que ladite poudre est additionnée d'eau.
